# EUROPEAN PATENT APPLICATION

(11) **EP 3 494 897 A1**
(43) Date of publication of application: **12.06.2019**
(21) Application number: 18210972.8
(22) Date of filing: 07.12.2018
(51) Int. Cl.: A61B 17/064

(54) **CHISEL CUT STAPLES FOR USE IN SURGICAL STAPLERS AND A METHOD FOR MANUFACTURING SURGICAL STAPLES**

(30) Priority: 08.12.2017 US 201762596353 P; 13.11.2018 US 201816189509
(71) Applicant: Covidien LP, Mansfield, MA 02048 (US)
(72) Inventor: PENNA, Christopher P., Guilford, Connecticut 06437 (US); KOLLAR, Charles R., West Hartford, Connecticut 06119 (US); MOZDZIERZ, Patrick, Glastonbury, Connecticut 06033 (US); JOYCE, Steven H, Wallingford, Connecticut 06492 (US); GUERRERA, Joseph M., Watertown, Connecticut 06795 (US)
(74) Representative: Maschio, Antonio

(57) **Abstract**

A surgical staple includes first and second legs that are interconnected by a backspan. Each of the first and second legs defines a longitudinal leg axis and extends from the backspan along the leg axis to a tip. The tip is sharpened such that the tip is symmetrical about a longitudinal leg plane that includes the leg axis. In addition, a method of manufacturing a surgical staple includes sharpening a tip of a leg of the surgical staple such that the tip is symmetrical about a longitudinal leg axis that includes a longitudinal leg axis of the leg.

## Description

### CROSS-REFERENCE TO RELATED APPLICATION

This application claims the benefit of and priority to U.S. Provisional Patent Application Serial No. 62/596,353, filed December 8, 2017, the entire disclosure of which is incorporated by reference herein.

### BACKGROUND

### 1. Technical Field

The present disclosure relates to surgical staples and, more specifically, to surgical staples having staple legs with chisel cut tips and methods for manufacturing such surgical staples.

### 2. Discussion of Related Art

Surgical stapling apparatus are employed by surgeons to sequentially or simultaneously apply one or more rows of staples to body tissue for the purpose of joining segments of body tissue together. Typically, a staple has a backspan and a leg extending away from each end of the backspan in a substantially orthogonal direction. Each staple leg has a free end spaced from the backspan. The free end of each leg has a tip which is sharpened to penetrate tissue and guide the respective leg through the tissue.

When the stapler is actuated, or "fired", a drive member ejects or pushes staples from a staple cartridge towards an anvil. As the staples are pushed towards the anvil, the tips of each staple penetrate tissue and guide the legs through the tissue. As the tips of the staples contact the anvil, the tips are deflected such that the legs of the staples are formed into a desired shape.

In some surgical procedures, the staple legs are malformed when the tips of the staple are deflected by the anvil. This can be a result of the tips veering off target as the tips pass through tissue and/or when the tips contact the anvil.

Accordingly, there is a continuing need in the surgical arts to reduce the number of malformed staples during a surgical procedure.

### SUMMARY

This disclosure generally relates to staples that have symmetrical tips which improve performance of staples during a surgical procedure. Specifically, the symmetrical tips improve tracking of the staple through tissue and improve the formation of staples when the tips contact an anvil.

This disclosure also relates to a method of forming staples with symmetrical tips. Specifically, this disclosure relates to a process of sharpening the tips of a staple with a "chisel-cut" such that the tips of the staple are sharpened in a symmetrical manner.

In an aspect of the present disclosure, a surgical staple includes first and second legs that are interconnected by a backspan. Each of the first and second legs defines a longitudinal leg axis and extends from the backspan along the leg axis to a tip. The tip is sharpened such that the tip is symmetrical about a longitudinal axial leg plane which includes the leg axis.

In aspects, the tip includes a face that defines an angle with the leg axis. The angle may be in a range of about 15° to about 60°. The face may have a substantially elliptical shape. The major axis of the face may be disposed at an angle with the leg axis and a minor axis of the face may be perpendicular to the leg axis. The minor axis of the face may be perpendicular to a longitudinal axis of the backspan.

In some aspects, the face includes a leading edge that is parallel to a minor axis of the face and is intersected by a major axis of the face. The leading edge may be configured to steer the tip through tissue.

In another aspect of the present disclosure, a method of manufacturing a surgical staple includes sharpening a tip of a leg of the surgical staple such that the tip is symmetrical about a longitudinal axial leg plane that includes a longitudinal leg axis of the leg.

In aspects, sharpening the tip of the leg includes passing a die through the tip to form a planar face on the tip that has a substantially elliptical shape. Passing the die through the tip includes moving the die in a direction along a major axis of the face. Moving the die in a direction along the major axis of the face may include moving the die along the face from a trailing edge of the face towards a leading edge of the face. Passing the die through the tip may include moving the die in a direction that defines an angle with the leg axis in a range of about 15° to about 60°.

In another aspect of the present disclosure, a surgical stapler includes a handle and a loading unit. The loading unit is releasably coupled to the handle. The loading unit includes an end effector having a staple cartridge that houses a plurality of staples. Each staple has first and second legs that are interconnected by a backspan. Each of the first and second legs defines a longitudinal leg axis and extends from the backspan along the leg axis to a tip. The tip is sharpened such that the tip is symmetrical about a longitudinal axial leg plane that includes the leg axis.

In aspects, the tip includes a face that defines an angle with the leg axis. The angle may be in a range of about 15° to about 60°.

In some aspects, the end effector assembly is selected from the group consisting of a linear end effector assembly, a circular end effector assembly, a transverse end effector assembly, and a curved end effector assembly. The handle may be configured to manually actuate the end effector assembly. The handle may be configured to electromechanically actuate the end effector assembly.

Further, to the extent consistent, any of the aspects described herein may be used in conjunction with any or all of the other aspects described herein.

### BRIEF DESCRIPTION OF THE DRAWINGS

Various aspects of the present staples are described hereinbelow with reference to the drawings, which are incorporated in and constitute a part of this specification, wherein:
FIG. 1 is a perspective view of a manually actuated circular stapler useful in applying staples provided in accordance with the present disclosure to tissue;
FIG. 2 is an enlarged perspective view of an end effector assembly of the circular stapler of FIG. 1 with an assembly of the end effector assembly separated from a loading unit of the end effector assembly;
FIG. 3 is a perspective view, with parts separated, of a portion of the loading unit shown in FIG. 2;
FIG. 4 is a perspective view of a manually actuated linear stapler useful in applying staples provided in accordance with the present disclosure to tissue;
FIG. 5 is a perspective view, with parts separated, of the end effector assembly of the linear stapler of FIG. 4;
FIG. 6 is a perspective view of an electromechanical surgical system and a plurality of loading units that are selectively attachable to a handle of the surgical system useful in applying staples provided in accordance with the present disclosure to tissue;
FIG. 7 is a perspective view of a prior art staple and a die for forming tips of the staple;
FIG. 8 is an enlarged view of the indicated area of detail of FIG. 8;
FIG. 9 is a side view of a tip of the prior art staple of FIG. 7;
FIG. 10 is a perspective view of the tip of the prior art staple of FIG. 7;
FIG. 11 is a perspective view of a staple and a die for forming tips of staples provided in accordance with the present disclosure;
FIG. 12 is an enlarged view of the indicated area of detail of FIG. 11;
FIG 13 is a side view of a tip of the staple of FIG. 11;
FIG. 14 is a perspective view of the tip of the staple of FIG. 11; and
FIG. 15 is a plan view of a tissue contacting surface of an anvil of the circular stapler instrument of FIG. 1.

### DETAILED DESCRIPTION

Embodiments of the present staples are now described in detail with reference to the drawings in which like reference numerals designate identical or corresponding elements in each of the several views. As used herein, the term "clinician" refers to a doctor, a nurse, or any other care provider and may include support personnel. Throughout this description, the term "proximal" refers to the portion of the device or component thereof that is closest to the clinician and the term "distal" refers to the portion of the device or component thereof that is farthest from the clinician.

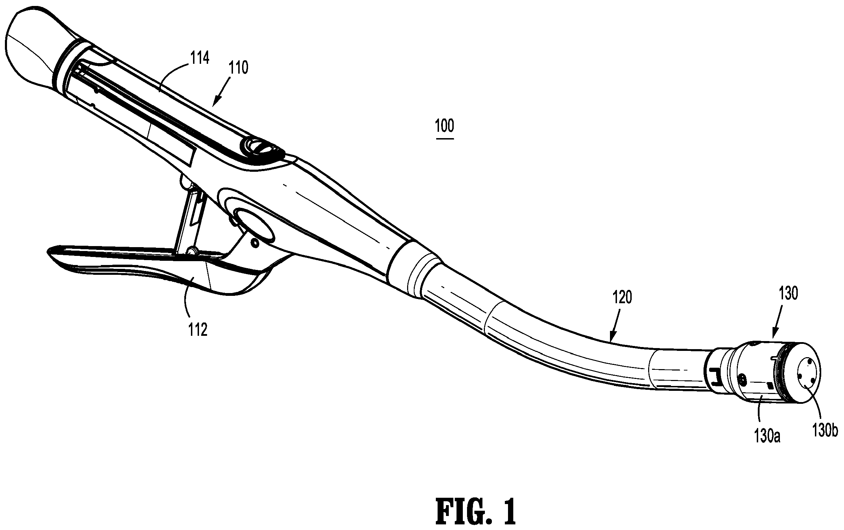

With reference to FIG. 1, a circular surgical stapler 100 useful in applying staples in accordance with the present disclosure includes a handle assembly 110, an elongate portion 120 extending distally from the handle assembly 110, and an end effector 130 secured to a distal portion of the elongate portion 120. The end effector 130 includes a loading unit 140 and an anvil 160. The handle assembly 110 has a moveable handle 112 and a fixed handle 114. The moveable handle 112 is movable towards the fixed handle 114 to actuate or fire the loading unit 130. For a detailed description of a similar circular surgical stapler, reference can be made to U.S. Patent No. 8,789,737 ("the '737 Patent"), the entire contents of which are hereby incorporated by reference.

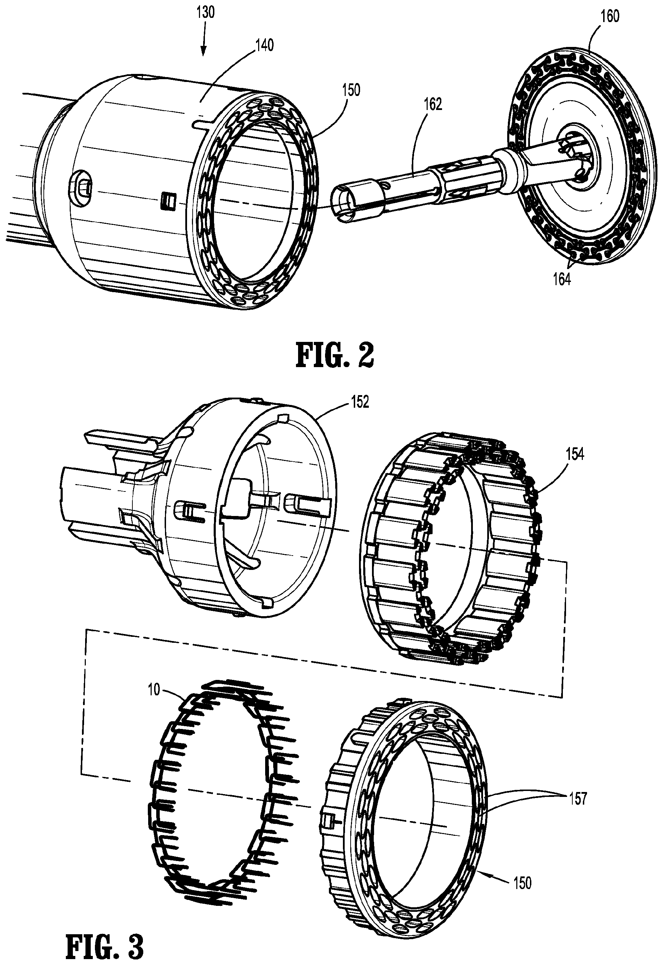

Referring to FIGS. 2 and 3, the loading unit 140 includes a staple cartridge 150 pusher driver 152, an annular ring of pushers 154, and staples 10 supported within the staple cartridge 150. The pusher driver 152 is positioned in abutting relation to a proximal end of the pushers 154. The staples 10 are supported within the staple cartridge 150 and are arranged in one or more rings with a pusher 154 of the annular ring of pushers 154 associated with one or more of each of the staples 10. The staple cartridge 150 defines a plurality of openings 157 that receive with the staples 10 and the pushers 154 such that as the loading unit 130 is fired, the pushers 154 eject or push the staples 10 towards the anvil 160 through the openings 157.

The anvil 160 includes a shaft 162 that passes through the loading unit 140 and is secured to the elongate portion 120 (FIG. 1) such that the handle assembly 110 draws the anvil assembly 160 towards the staple cartridge 150 when the handle assembly 110 is actuated to fire the staples 10 such as described in more detail in the '737 Patent. The anvil 160 also defines staple pockets 164 on a tissue contacting surface of the anvil 160 that is opposed to the staple cartridge 150. Each staple pocket 164 is aligned with a respective staple 10 when the anvil 160 is secured to the elongate portion 120 such that when the staples 10 are pushed towards the anvil 160, each staple 10 is received within a respective staple pocket 164 such that the staple 10 is formed in tissue disposed between the loading unit 140 and the anvil assembly 160.

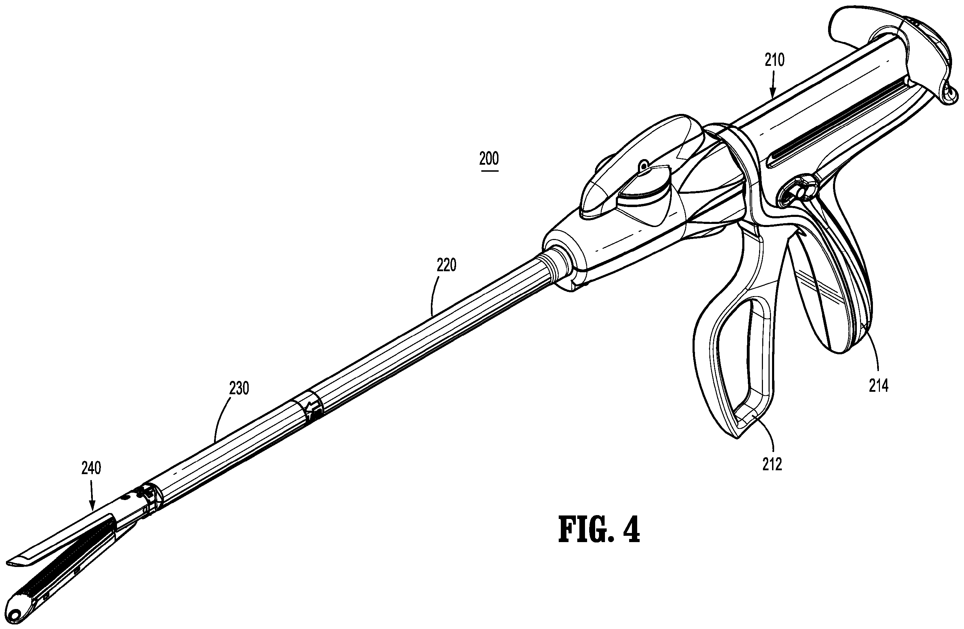

Referring to FIG. 4, a linear surgical stapler 200 useful in applying staples in accordance with the present disclosure includes a handle assembly 210, an elongate portion 220 extending distally from the handle assembly 210, and a loading unit 230 secured to a distal portion of the elongate portion 220. The loading unit 230 includes an end effector assembly 240. The handle assembly 210 has a moveable handle 212 and a fixed handle 214. In embodiment, the moveable handle 212 is movable towards the fixed handle 214 to move the end effector assembly 240 towards a clamped position and actuate or fire end effector assembly 240 of the loading unit 230. For a detailed description of an exemplary linear surgical stapler, reference can be made to U.S. Patent Publication No. 2015/0297216, the entire contents of which are hereby incorporated by reference. Alternatively, the stapler 200 may include separate handles for effecting clamping and firing of the stapler 200.

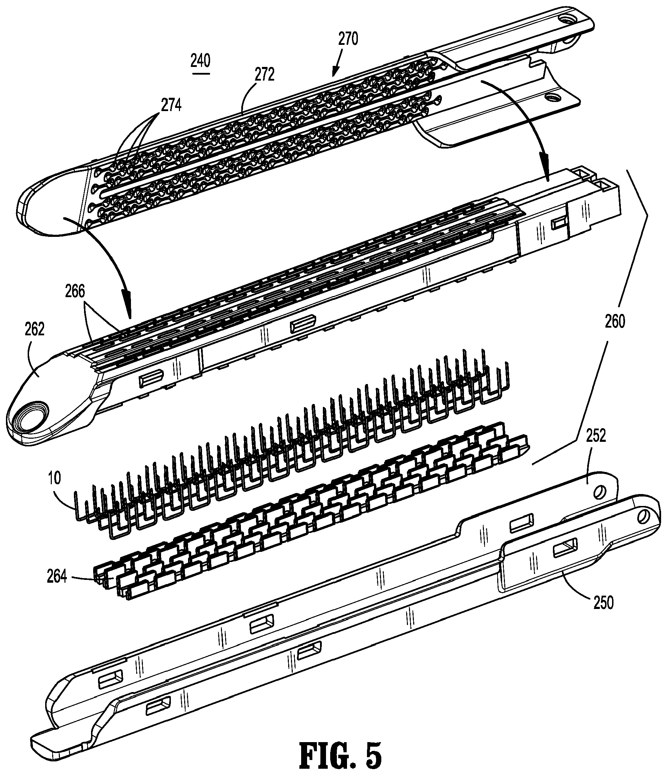

Referring to FIG. 5, the end effector assembly 240 includes a first jaw 250 defining a channel 252, a staple cartridge 260 releasably received within the channel 252, and a second jaw 270 having an anvil 272 defining staple pockets 274. The staple cartridge 250 includes a body 262, pushers 264, and staples 10. In embodiments, the staples 10 are arranged in one or more rows with one of the pushers 264 associated with one or more of the staples 10. The body 262 defines a plurality of openings 262 that receive the staples 10 and the pushers 264 such that as the end effector assembly 240 is fired, the pushers 264 eject or push the staples 10 through the openings 262 towards the anvil 272. As the each staple 10 is received within a respective staple pocket 274 of the anvil 272, the staple 10 is formed in tissue disposed between the staple cartridge 260 and the anvil 272.

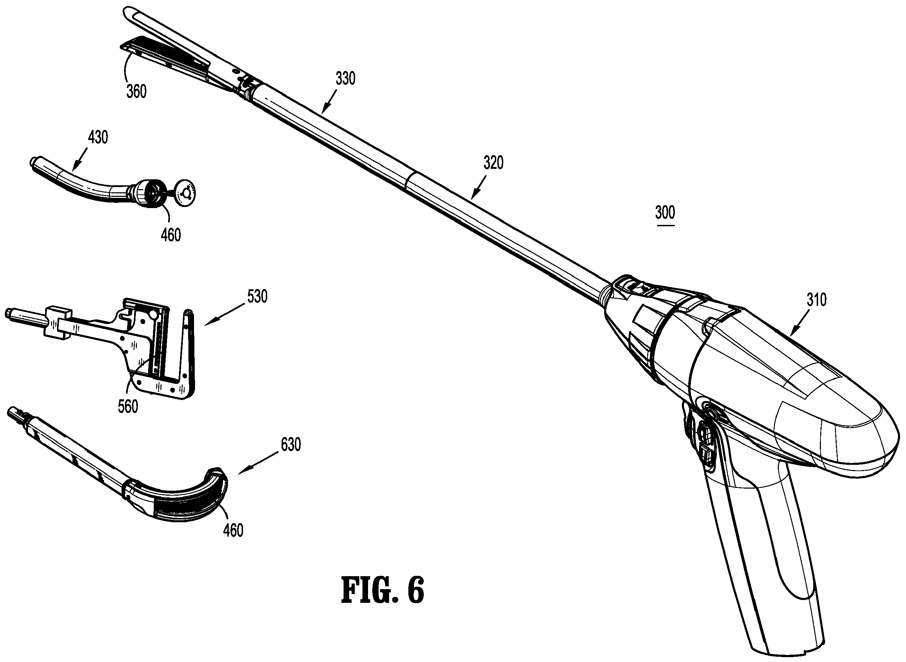

Referring to FIG. 6, an electromechanical surgical instrument or system 300 useful in applying staples in accordance with the present disclosure includes a powered handle 310, an adapter 320, and one or more loading units 330, 430, 530, 630 which may be selectively attached to the adapter 320. The loading unit may include, but is not limited to a linear loading unit 330, a circular loading unit 430, a transverse loading unit 530, or a curved loading unit 630. Each of the loading units 330, 430, 530, 630 includes a staple cartridge 360, 460, 560, 660 having a plurality of staples, e.g., staples 10 (FIG. 3). For a detailed description of an exemplary electromechanical surgical system 300 reference can be made to U.S. Patent Publication No. 2016/0310134, the entire contents of which are hereby incorporated by reference.

In addition, it is contemplated that the staples 10, detailed below, may be used with a loading unit secured to a robotic surgical system such that the robotic surgical system actuates an end effector assembly of the loading unit to clamp tissue and/or to fire staples from the loading unit. An exemplary robotic surgical system is disclosed in U.S. Patent Nos. 8,828,023 and 9,301,691, the entire contents of which are incorporated herein by reference.

The above surgical instrument, e.g., instruments 100, 200, and electromechanical surgical system 300 are meant to be exemplary surgical instruments and systems which may include one or more of the staples 10 detailed below. For example, the staple 10 may also be used with a transverse stapling instrument and/or a manually actuated curved stapling instrument.

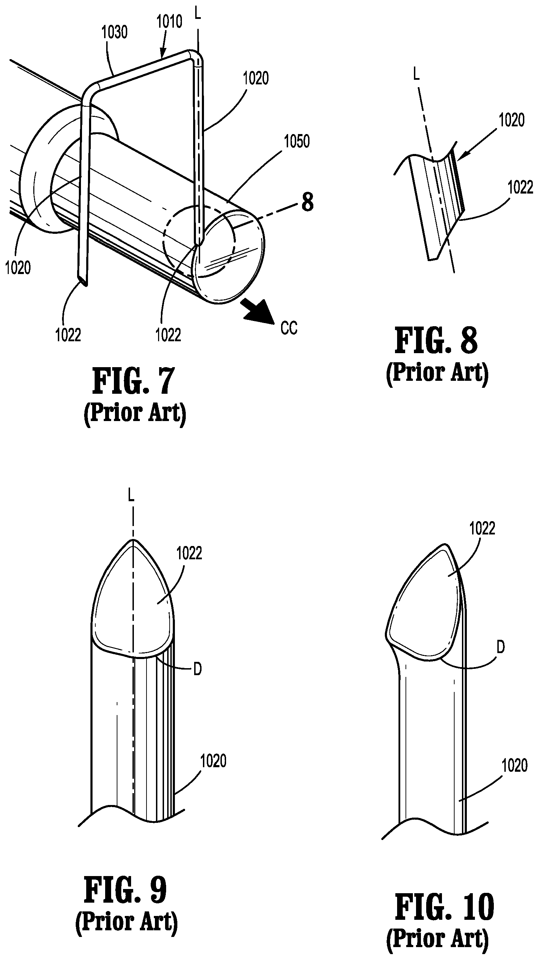

With reference to FIGS. 7-10, a prior art staple 1010 and a method of sharpening legs of the prior art staple 1010 are described in an effort to illustrate the advantages of the method of sharpening the legs of the presently disclosed staples 10. With particular reference to FIG. 7, the staple 1010 includes legs 1020 interconnected by a backspan 1030. Each leg 1020 defines a longitudinal leg axis L and has a tip 1022 that is spaced apart from the backspan 1030. Each of the tips 1022 is sharpened to a tapered point that allows the tip 1022 to pierce tissue and to direct the legs 1020 toward an anvil, e.g., anvil 160 (FIG. 3), such that the staple 1010 is properly formed upon actuation of an end effector assembly, e.g., end effector assembly 130 (FIG. 3).

Generally, the tip 1022 is formed using a cross-cut process. In a cross-cut process, a die tool 1050 is punched or extended through the tip 1022 of each of the legs 1020 of the staples 1010 in a direction perpendicular to the longitudinal leg axis L of the staple leg 1020 as shown by the arrow CC in FIG. 7. Engagement of the die tool 1050 and the tips 1022 of the legs 1020 forms a tapered tip 1022.

With particular reference to FIGS. 9 and 10, the cross-cutting process forms a tip 1022 with is not symmetrical in shape about the leg axis L. Specifically, as the die 1050 cuts the tip 1022, material of the tip 1022 is moved in the direction of travel of the die 1050, as shown by arrow CC (FIG. 7). This may result in distortion D on the tip 1022. This distortion D has been shown to steer the staple legs 1020 off target as the tip 1022 travels through tissue, especially in thick tissue indications, and towards an anvil assembly.

In several test firings of a circular stapling cartridge with only a single ring of staples 1010 loaded in the circular stapling cartridge, e.g., cartridge 150 (FIG. 3), there was an average of about 5 malformed staples for each firing of the cartridge. These malformed staples may be a result of a distorted tip 1022 steering the leg 1020 of the prior art staple 1010 out of alignment with the anvil assembly as the tip 1022 travels through tissue.

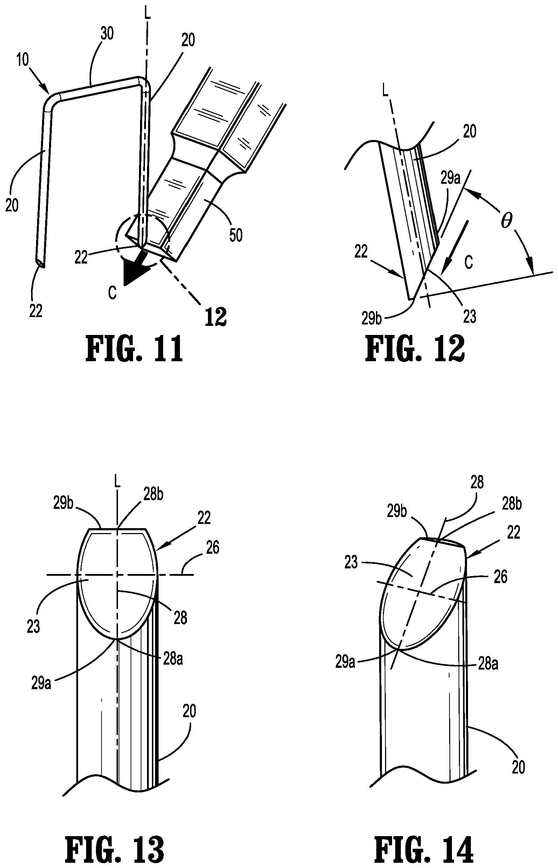

Referring now to FIGS. 11-14, the staple 10 and a method of sharpening and/or cutting a tip 22 of the legs 20 of the staple 10 is disclosed in accordance with the present disclosure. The staple 10 has legs 20 interconnected by a backspan 30. Each of the legs 20 defines a longitudinal leg axis L and has a tip 22 spaced apart from the backspan 30. The tip 22 has a planar face 23 that is generally elliptical in shape with a minor axis 26 that is perpendicular to both the longitudinal leg axis L and a longitudinal axis of the backspan 30 and a major axis 28 defining an angle θ relative to the longitudinal leg axis L in a range of about 15° to about 85°, in embodiments about 35°. The major axis intersects the longitudinal leg axis L between the foci of the planar face 23 and intersects an edge of the planar face 23 at a trailing point 28a and a leading point 28b with the trailing point 28a being closer to the backspan 30 along the longitudinal leg axis L than the leading point 28b. The planar face 23 includes a trailing edge 29a adjacent the trailing point 28a and a leading edge 29b adjacent the leading point 28b. The leading edge 29b may be linear and define an axis that is parallel to the minor axis 26 with the leading point 28b being positioned substantially at a center point of the leading edge 29b. The leading edge 29 is configured to penetrate tissue and to steer the tip 22 through tissue.

To sharpen or cut the tips 22 of the staple 10 by a chisel cut process, the die 50 is moved along the major axis 28 in a direction parallel to a face 23 of the respective tip 22 which is represented by arrow C. As shown, the die 50 is moved along the major axis 28 from the trailing edge 29a towards the leading edge 29b; however, the die 50 may be moved along the major axis 28 from the leading edge 29b towards the trailing edge 29a. By sharpening the tip 22 by the chisel cut process, the tip 22 when formed, is symmetrical about a longitudinal axial leg plane which includes the longitudinal leg axis L.

Because the tip 22 is symmetrical, the tip 22 is better able to travel straight through tissue without veering off target. By traveling straight through tissue, the accuracy of the tip 22 striking the anvil is increased by an order of magnitude when compared to the prior art staples 1010. For example, in testing, the tip 22 of the staple legs 20 struck an anvil, e.g., anvil 160 (FIG. 3), within about ± 0.001 inches of a target area. In contrast, the tips 1022 of the prior art staples 1010 struck an anvil within about ± 0.01 inches of a target area. The increase in accuracy may also improve staple formation, especially in thick tissue procedures, when compared to the prior art staples 1010 where the distortions D of the prior art staples 1010 (FIGS. 9 and 10) may steer the tip 1022, and thus the leg 1020, off target which can lead to malformed staples. In testing, the number of malformed staples with the staple 10 was reduced to an average of about 0 malformed staples for each firing of the cartridge when compared to an average of about 5 malformed staples for similar firings of the prior art staples 1010 as detailed above.

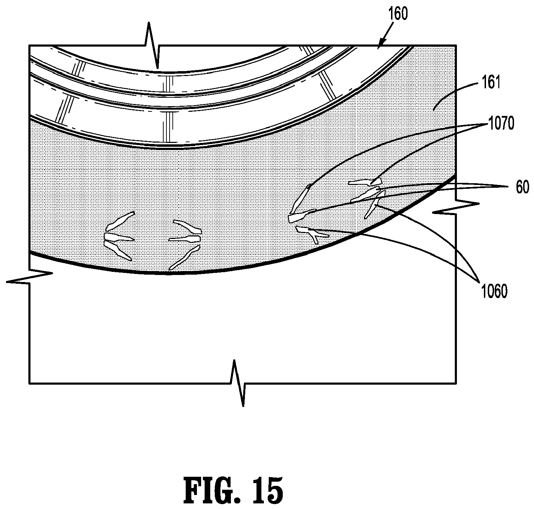

With reference to FIG. 15, strike patterns of the prior art staple 1010 and the staple 10 are shown on the tissue contacting surface 161 of the anvil 160 of the circular stapling instrument 100. The strike pattern of the prior art staple 1010 is dependent on the direction that the staple 1010 is loaded in the cartridge 150 (FIG. 3). For example, when the staple 1010 is loaded in a first direction with the distortion D (FIGS. 9 and 10) positioned towards the outside of the anvil 160, the staple 1010 tracks away from the center of the anvil 160 as the tip 1022 of the prior art staple 1010 passes through tissue to form strikes 1060. Alternatively, when the staple 1010 is loaded in a second direction with the distortion D positioned towards the inside of the anvil 160, the staple 1010 tracks towards the center of the anvil 160 as the tip 1022 of the prior art staple 1010 passes through tissue to form strikes 1070. In contrast, when the tip 22 of the staples 10 passes through tissue, the tip 22 stays straight and contacts the tissue contacting surface 161 of the anvil 160 such that the tip 22 tracks directly towards the other tip 22 as shown by the track 60. By having a predictable and repeatable track, e.g., track 60, the likelihood of malformed staples is reduced when using staple 10 when compared to staple 1010.

While several embodiments of the disclosure have been shown in the drawings, it is not intended that the disclosure be limited thereto, as it is intended that the disclosure be as broad in scope as the art will allow and that the specification be read likewise. Any combination of the above embodiments is also envisioned and is within the scope of the appended claims. Therefore, the above description should not be construed as limiting, but merely as exemplifications of particular embodiments. Those skilled in the art will envision other modifications within the scope of the claims appended hereto.

The invention may be described by reference to the following numbered paragraphs:-
1. A surgical staple comprising:
   first and second legs interconnected by a backspan, each of the first and second legs defining a longitudinal leg axis and extending from the backspan along the leg axis to a tip, the tip being sharpened such that the tip is symmetrical about a longitudinal leg plane that includes the leg axis.
2. The staple according to paragraph 1, wherein the tip includes a face that defines an angle with the leg axis.
3. The staple according to paragraph 2, wherein the angle is in a range of 15° to 60°.
4. The staple according to paragraph 2, wherein the face has a substantially elliptical shape.
5. The staple according to paragraph 4, wherein a major axis of the face is disposed at the angle with the leg axis and a minor axis of the face is perpendicular to the leg axis.
6. The staple according to paragraph 5, wherein the minor axis of the face is perpendicular to a longitudinal axis of the backspan.
7. The staple according to paragraph 2, wherein the face includes a leading edge that is parallel to a minor axis of the face and is intersected by a major axis of the face, the leading edge being configured to steer the tip through tissue.
8. A method of manufacturing a surgical staple, the method comprising:
   sharpening a tip of a leg of the surgical staple such that the tip is symmetrical about a longitudinal leg plane that includes a longitudinal leg axis of the leg.
9. The method according to paragraph 8, wherein sharpening the tip of the leg includes passing a die through the tip to form a planar face on the tip having a substantially elliptical shape.
10. The method according to paragraph 9, wherein passing the die through the tip includes moving the die in a direction along a major axis of the face.
11. The method according to paragraph 10, wherein moving the die in a direction along the major axis of the face includes moving the die along the face from a trailing edge of the face towards a leading edge of the face.
12. The method according to paragraph 9, wherein passing the die through the tip includes moving the die in a direction defining an angle with leg axis in a range of 15° to 60°.
13. A surgical stapler comprising:
   a handle; and
   a loading unit releasably coupled to the handle, the loading unit including an end effector having a staple cartridge, the staple cartridge housing a plurality of staples, each staple having first and second legs interconnected by a backspan, each of the first and second legs defines a longitudinal leg axis and extends from the backspan along the leg axis to a tip, the tip being sharpened such that the tip is symmetrical about a longitudinal leg plane that includes the leg axis.
14. The surgical stapler according to paragraph 13, wherein the tip includes a face that defines an angle with the leg axis.
15. The surgical stapler according to paragraph 14, wherein the angle is in a range of 15° to 60°.
16. The surgical stapler according to paragraph 13, wherein the end effector assembly is selected from the group consisting of a linear end effector assembly, a circular end effector assembly, a transverse end effector assembly, and a curved end effector assembly.
17. The surgical stapler according to paragraph 13, wherein the handle is configured to manually actuate the end effector assembly.
18. The surgical stapler according to paragraph 13, wherein the handle is configured to electromechanically actuate the end effector assembly.

## Claims

1. A surgical staple comprising:
first and second legs interconnected by a backspan, each of the first and second legs defining a longitudinal leg axis and extending from the backspan along the leg axis to a tip, the tip being sharpened such that the tip is symmetrical about a longitudinal leg plane that includes the leg axis.

2. The staple according to claim 1, wherein the tip includes a face that defines an angle with the leg axis.

3. The staple according to claim 2, wherein the angle is in a range of 15° to 60°.

4. The staple according to claim 2 or claim 3, wherein the face has a substantially elliptical shape.

5. The staple according to claim 4, wherein a major axis of the face is disposed at the angle with the leg axis and a minor axis of the face is perpendicular to the leg axis.

6. The staple according to claim 5, wherein the minor axis of the face is perpendicular to a longitudinal axis of the backspan.

7. The staple according to claim 2, wherein the face includes a leading edge that is parallel to a minor axis of the face and is intersected by a major axis of the face, the leading edge being configured to steer the tip through tissue.

8. A method of manufacturing a surgical staple, the method comprising:
sharpening a tip of a leg of the surgical staple such that the tip is symmetrical about a longitudinal leg plane that includes a longitudinal leg axis of the leg.

9. The method according to claim 8, wherein sharpening the tip of the leg includes passing a die through the tip to form a planar face on the tip having a substantially elliptical shape.

10. The method according to claim 9, wherein passing the die through the tip includes moving the die in a direction along a major axis of the face.

11. The method according to claim 10, wherein moving the die in a direction along the major axis of the face includes moving the die along the face from a trailing edge of the face towards a leading edge of the face.

12. The method according to claim 9, wherein passing the die through the tip includes moving the die in a direction defining an angle with leg axis in a range of 15° to 60°.

13. A surgical stapler comprising:
a handle; and
a loading unit releasably coupled to the handle, the loading unit including an end effector having a staple cartridge, the staple cartridge housing a plurality of staples, each staple having first and second legs interconnected by a backspan, each of the first and second legs defines a longitudinal leg axis and extends from the backspan along the leg axis to a tip, the tip being sharpened such that the tip is symmetrical about a longitudinal leg plane that includes the leg axis.

14. The surgical stapler according to claim 13, wherein the tip includes a face that defines an angle with the leg axis; preferably wherein the angle is in a range of 15° to 60°; and/or wherein the end effector assembly is selected from the group consisting of a linear end effector assembly, a circular end effector assembly, a transverse end effector assembly, and a curved end effector assembly.

15. The surgical stapler according to claim 13, wherein the handle is configured to manually actuate the end effector assembly; or wherein the handle is configured to electromechanically actuate the end effector assembly.
